# EUROPEAN PATENT APPLICATION

(11) **EP 4 445 844 A1**
(43) Date of publication of application: **16.10.2024**
(21) Application number: 22902695.0
(22) Date of filing: 24.04.2022
(51) Int. Cl.: A61B 5/296, A61B 5/268

(54) **FLEXIBLE ELECTROMYOGRAPHIC ELECTRODE ARRAY, AND PREPARATION METHOD THEREFOR AND APPLICATION THEREOF**

(30) Priority: 09.12.2021 CN 202111500574
(71) Applicant: Southern University of Science and Technology, Shenzhen, Guangdong 518000 (CN)
(72) Inventor: JIANG, Xingyu, Shenzhen, Guangdong 518000 (CN); YANG, Shuaijian, Shenzhen, Guangdong 518000 (CN); CHAKRABARTY, Samit, Shenzhen, Guangdong 518000 (CN)
(74) Representative: Klunker IP Patentanwälte PartG mbB
(86) International application number: PCT/CN2022/088644
(87) International publication number: WO 2023/103255

(57) **Abstract**

A flexible electromyographic electrode array and a preparation method therefor, and an electromyographic signal collection apparatus and an electrophysiological signal collection method. The flexible electromyographic electrode array comprises: a flexible substrate (100); several liquid metal electrodes, wherein the liquid metal electrodes are arranged on the flexible substrate (100), and the liquid metal electrodes each comprise a wire and a circuit board connection pattern (110) which is located at one end of a wire (120); and several electrode contacts (130), wherein each of the electrode contacts (130) is connected to the other end of the wire (120), and the electrode contacts (130) are made of a contact solution which contains a hydrophilic polymer and a poly(3,4-ethylenedioxythiophene)/polystyrene sulfonate. The preparation method comprises: printing liquid metal ink onto a substrate material, so as to form a metal electrode pattern; transfer-printing the electrode pattern onto a flexible substrate, so as to form several metal electrodes; and performing plasma cleaning by means of a mask, and dripping the raw material of electrode contacts (130), so as to form the electrode contacts (130). The electromyographic signal collection apparatus comprises a flexible electromyographic electrode array. The electrophysiologic signal collection method comprises: covering the skin surface of a subject with a flexible electromyographic electrode array, and acquiring an electromyographic signal. The electrode contacts have good biocompatibility, high conductivity and low impedance, the tight attachment between electrodes and skin can be realized by means of the electrode contacts, and the impedance of the electrode contacts (130) does not increase due to the loss of moisture, such that the quality of signal recording is prevented from being affected by the generation of noise.

## Description

### TECHNICAL FIELD

The present disclosure relates to the technical field of electrode arrays, and in particular to a flexible electromyographic electrode array, and a preparation method therefor and an application thereof.

### BACKGROUND

Electromyography (EMG) is one of medical diagnosis and treatment technologies for recording and evaluating an electrical signal generated from skeletal muscle. The functional status of the muscle can be effectively reflected by extracting and analyzing characteristic information in the electromyographic signal, and thus EMG is widely used in many fields such as medical diagnosis, post-injury rehabilitation, sports science and ergonomics. In an actual practice, the collection of the electromyographic signal usually requires the cooperation of electromyographic electrode, amplifier, filter and monitor.

Currently, the electrode used for the electromyographic signal collection can be mainly divided into two types: needle electromyographic electrode and surface electromyographic electrode. The needle electromyographic electrode is required to be pierced into the muscle through the skin when used, so that the electromyographic signal of a single motor unit can be collected, and thus the needle electromyographic electrode is usually used for medical diagnosis. However, as an invasive means, the needle electromyographic electrode may cause pain to the subject when being pierced into the skin. Meanwhile, the operation of professional medical staff is required, and the accuracy of the measurement depends on the needle entering depth. In comparison, the surface electromyographic electrode can serve as a non-invasive detection means due to the attachment to the surface of the skin during use, no damage to the epidermis and convenient measurement. Most of the traditional surface electrodes use a metal electrode as a contact. However, the metal electrode is prone to produce serious noise in the detection process, thus affecting the signal recording. Therefore, it is necessary to provide an electromyographic electrode array capable of recording a high-quality electromyographic signal.

### SUMMARY

The present disclosure intends to solve at least one of the technical problems existing in the prior art. In view of this, the present disclosure proposes an electromyographic electrode array capable of recording a high-quality electromyographic signal, and a preparation method therefor and an application thereof.

In a first aspect of the present disclosure, provided is a flexible electromyographic electrode array, which includes:
a flexible substrate;
several liquid metal electrodes provided on the flexible substrate, wherein each of the liquid metal electrodes includes a wire and a circuit board connection pattern disposed at an end of the wire; and
several electrode contacts provided on the flexible substrate, wherein each of the electrode contacts is connected to the other end of the wire, and raw material of the electrode contact includes a hydrophilic polymer and poly(3,4-ethylenedioxythiophene)/polystyrene sulfonate (PEDOT: PSS).

The flexible electromyographic electrode array according to the embodiment of the present disclosure has at least the following beneficial effects.

The applicant found that the reason why the serious noise is easily produced when using a metal electrode as a contact during the detection is that, when a deformation of the skin or muscle occurs, gaps may form between the electrode contact and the skin due to the lack of flexibility in the material and no assurance of a tight attachment between the electrode and the skin, thereby creating additional capacitance and resistance, increasing the overall circuit impedance and causing serious noise. The flexible electromyographic electrode array provided in the present disclosure uses a flexible material as the substrate, a liquid metal as the main part of the electrode material, and a combination of the hydrophilic polymer and poly(3,4-ethylenedioxythiophene)/polystyrene sulfonate as the raw material of the electrode contact. The wire, electrode and substrate are all of extremely flexible material, thus providing an outstanding advantage in flexibility. In addition, the particular combination of the hydrophilic polymer and poly(3,4-ethylenedioxythiophene)/polystyrene sulfonate as the electrode contact has the effects of good biocompatibility, high conductivity, and low impedance. In this way, the electromyographic signal of a certain motor unit in the muscle can be detected by a separate electrode contact, and the flexibility of the material can enable the tight attachment between the electrode and the skin, without causing additional impedance due to the gaps, and without increasing the impedance of the electrode contact due to the loss of the moisture, thereby preventing the quality of signal recording from the generated serious noise.

In some embodiments of the present disclosure, a mass ratio of the hydrophilic polymer to the poly(3,4-ethylenedioxythiophene)/polystyrene sulfonate is 1: (0.1 to 4). Further, the mass ratio is 1: (0.2 to 2); further, the mass ratio is 1: (0.4 to 1); or further, the mass ratio is 1: (0.4 to 0.6). By further controlling the mass ratio of the hydrophilic polymer to the poly(3,4-ethylenedioxythiophene)/polystyrene sulfonate, the flexibility and conductivity of the electrode contact can be further improved, and the signal quality can be further enhanced.

In some embodiments of the present disclosure, the raw material of the electrode contact further includes at least one of dimethyl sulfoxide, ethylene glycol, and glycidyloxypropyltrimethoxysilane. The dimethyl sulfoxide, ethylene glycol, and glycidyloxypropyltrimethoxysilane are added as dopants to improve the final conductivity of the formed electrode contact and improve the signal quality. In some embodiments of the present disclosure, a volume percentage of the dopants in the raw material of the electrode contact is 1% to 10%.

In some embodiments of the present disclosure, the hydrophilic polymer is at least one selected from the group consisting of polyethylene glycol (PEG), polyethylene oxide (PEO), and polyvinyl alcohol (PVA). By combining at least one of the above three polymers with PEDOT: PSS, the electrode contact with good conductivity and flexibility can be obtained.

In some embodiments of the present disclosure, the flexible substrate is further provided with a skin adhesive layer. The applied flexible skin adhesive brings a good adhesion to the electromyographic electrode array, which can ensure continuous and stable contact of the skin and the flexible circuit board, thus ensuring the signal quality.

In some embodiments of the present disclosure, the flexible substrate is further provided with an insulating layer. The material of the insulating layer includes at least one of silicon nitride, polydimethylsiloxane, and SU-8 photoresist.

In some embodiments of the present disclosure, the insulating layer and the skin adhesive layer are sequentially provided on the flexible substrate at positions other than the circuit board connection patterns and the electrode contacts.

In some embodiments of the present disclosure, a spacing between the electrode contacts is 3 mm to 50 mm.

In some embodiments of the present disclosure, a size of the electrode contact is 1 mm to 10 mm. By controlling the size of electrode contact and the spacing between the electrode contacts, signal crosstalk between the adjacent electrode contacts can be avoided, and the signal collected therefrom is more accurate.

In some embodiments of the present disclosure, a size of the circuit board connection pattern is 1 mm to 10 mm.

In some embodiments of the present disclosure, a width of the wire is 50 mm to 1000 mm.

In a second aspect of the present disclosure, provided is a preparation method for the above flexible electromyographic electrode array. The preparation method includes the following steps:
(1) coating a liquid metal ink onto a substrate material to form a liquid metal electrode pattern, wherein the liquid metal electrode pattern includes a wire, a circuit board connection pattern disposed at an end of the wire, and an electrode connection pattern disposed at the other end of the wire;
(2) transfer-printing the liquid metal electrode pattern onto a flexible substrate to form several liquid metal electrodes; and
(3) performing plasma cleaning on the electrode connection pattern by a mask plate to change hydrophilicity or hydrophobicity of position of the electrode connection pattern, and dripping raw material of electrode contact to form several electrode contacts.

In some embodiments of the present disclosure, the method further includes the following step: (4) covering the electrode contacts and the circuit board connection pattern with a baffle, performing encapsulation, and removing the baffle.

In some embodiments of the present disclosure, the step (4) includes: covering the electrode contact and the circuit board connection pattern with the baffle, performing primary encapsulation using an insulating material, performing secondary encapsulation using a skin adhesive, and removing the baffle.

In some embodiments of the present disclosure, the step (4) includes: covering the electrode contact and the circuit board connection pattern with the baffle, performing spin coating and encapsulation using a PDMS solution (including PDMS and an appropriate amount of curing agent) with a rotation speed of 1000 to 3000 rpm for 10 to 60 seconds, drying after the completion of the spin coating to complete the primary encapsulation; then performing spin coating and encapsulation using a flexible skin adhesive with a rotation speed of 1000 to 3000 rpm for 10 to 60 seconds, and drying after the completion of the spin coating to complete the secondary encapsulation.

In a third aspect of the present disclosure, provided is an electromyographic signal collection apparatus, which includes the above flexible electromyographic electrode array.

In a fourth aspect of the present disclosure, provided is an electrophysiological signal collection method, which includes covering the above flexible electromyographic electrode array onto a skin surface of a subject, and acquiring an electromyographic signal.

Additional aspects and advantages of the present disclosure will be set forth in part in the description which follows, and in part will be obvious from the description or may be learned by practice of the present disclosure.

### BRIEF DESCRIPTION OF DRAWINGS

Figure 1 shows a schematic diagram of a flexible electromyographic electrode array according to an embodiment of the present disclosure.
Figure 2 shows a side view of a flexible electromyographic electrode array according to an embodiment of the present disclosure.
Figure 3 shows a schematic diagram of a pattern mask plate of a liquid metal electrode according to an embodiment of the present disclosure.
Figure 4 shows a schematic diagram of an electrode contact mask plate according to an embodiment of the present disclosure.
Figure 5 shows a photograph of a flexible electromyographic electrode array finally prepared in Example 1 of the present disclosure.
Figure 6 shows a photograph of a collected electromyographic signal of biceps brachii in Example 1 of the present disclosure.
Figure 7 shows a collected high-throughput electromyographic signal of biceps brachii in Example 1 of the present disclosure.
Figure 8 shows an electrical signal collected after 20Hz high-throughput filtering in a comparative experiment of the present disclosure, where panel a shows the result from Comparative Example 1, and panel b shows the result from Example 1.
Figure 9 shows a root mean square (RMS) value of noise (panel a) and a signal-to-noise ratio (SNR) (panel b) of the electrical signal shown in Figure 8.
Figure 10 shows a schematic principle diagram of noise generation during electrical signal detection shown in Figure 8, where panel a shows the principle for Comparative Example 1, and panel b shows the principle for Example 1.

Reference signs: flexible substrate 100, circuit board connection pattern 110, wire 120, electrode contact 130, insulating layer 210, skin adhesive layer 220, circuit board connection mask pattern 310, wire mask pattern 320, contact mask pattern 330, through hole 410.

### DETAILED DESCRIPTION

The concept of the present disclosure and the technical effects produced by the present disclosure will be clearly and completely described below in conjunction with the embodiments to fully understand the purpose, features and effects of the present disclosure. Obviously, the described embodiments are only some of the embodiments of the present disclosure, but not all. Based on the embodiments of the present disclosure, other embodiments obtained by those skilled in the art without exerting creative efforts are all within the protection scope of the present disclosure.

The embodiments of the present disclosure are described in detail below. The described embodiments are exemplary and only intended to explain the present disclosure and should not be understood as limiting the present disclosure.

In the description of the present disclosure, "several" refers to one or more, "multiple" refers to two or more, "greater than", "less than", "exceeding", etc. are understood to exclude the indicated number, and "above", "below", "within", etc. are understood to include the indicated number. If there is a description of "first" and "second", it is only for the purpose of distinguishing technical features, and should not be understood as indicating or implying the relative importance or implicitly indicating the number of indicated technical features or implicitly indicating the order of indicated technical features.

In the description of the present disclosure, the description of reference terms "an embodiment", "some embodiments", "an illustrative embodiment", "an example", "a specific example" or "some examples", etc. refer to specific feature, structure, material or characteristic described in conjunction with the embodiment or example and included in at least one embodiment or example of the present disclosure. In the description, schematic representations of the above terms do not necessarily refer to the same embodiment or example. Furthermore, the specific feature, structure, material or characteristic described may be combined in any suitable manner in any one or more embodiments or examples.

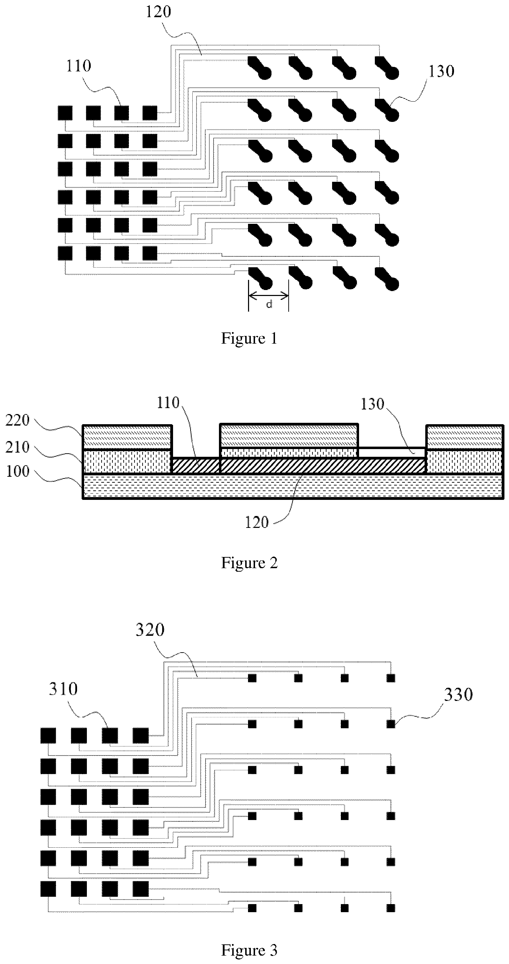

Referring to Figures 1 to 2, Figure 1 shows a schematic diagram of a flexible electromyographic electrode array according to an embodiment of the present disclosure, and Figure 2 shows a partial side view of the flexible electromyographic electrode array. The flexible electromyographic electrode array includes a flexible substrate 100; several liquid metal electrodes provided on the flexible substrate 100, wherein each of the liquid metal electrodes includes a wire 120 and a circuit board connection pattern 110 disposed at an end of the wire 120; and several electrode contacts 130 provided on the flexible substrate 100, wherein each of the electrode contacts 130 is connected to the other end of the wire 120. The electrode contact 130, the wire 120 and the circuit connection board pattern 110 constitute a complete electrical signal collection path. In some particular embodiments, the electrode contact 130, the wire 120 and the circuit connection board pattern 110 correspond to each other one-to-one, thereby forming several complete electrodes. Raw material of the electrode contact 130 include a hydrophilic polymer and poly(3,4-ethylenedioxythiophene)/polystyrene sulfonate. The substrate, wire, circuit board connection pattern and electrode contact of the flexible electromyographic electrode array are all made of flexible materials, thereby ensuring the overall flexibility. The hydrophilic polymer and poly(3,4-ethylenedioxythiophene)/polystyrene sulfonate applied in the electrode contact 130 bring a good flexibility and conductivity to the formed electrode contact, thus allowing a separate electrode contact to detect the electromyographic signal of a certain motor unit in the muscle. The flexibility of the material can also ensure the tight attachment between the electrode and the skin, without increasing the impedance of the electrode contact due to the loss of the moisture, thereby preventing the quality of signal recording from the generated noise.

In some particular embodiments, a mass ratio of the hydrophilic polymer to the poly(3,4-ethylenedioxythiophene)/polystyrene sulfonate in the raw material of the electrode contact is 1: (0.1 to 4). By controlling the ratio of the hydrophilic polymers to PEDOT: PSS, the flexibility and conductivity of the prepared electrode contact can be adjusted. In some preferred embodiments, the mass ratio is 1: (0.2 to 2); or further, the mass ratio is 1: (0.4 to 1), or 1: (0.4 to 0.6).

In some particular embodiments, the raw material of the electrode contact further includes at least one of dimethyl sulfoxide, ethylene glycol, and glycidyloxypropyltrimethoxysilane. The dimethyl sulfoxide, ethylene glycol, and glycidyloxypropyltrimethoxysilane are added as dopants in components of contact solution to improve the final conductivity of the formed electrode contact and improve the signal quality.

In some particular embodiments, the hydrophilic polymer is at least one selected from the group consisting of polyethylene glycol (PEG), polyethylene oxide (PEO), and polyvinyl alcohol (PVA). The combination of at least one of the above three polymers with PEDOT: PSS facilitates obtaining an electrode contact with good conductivity and flexibility.

In some particular embodiments, the material applied for the flexible substrate is at least one of the well-known flexible polymer materials with good biocompatibility in the art, including polydimethylsiloxane, polylactic acid, polyimide, poly(lactic acid-co-glycolic acid), and polycaprolactone. In some particular embodiments, a thickness of the flexible substrate is 50 µm to 200 µm.

In some particular embodiments, the material applied for the liquid metal electrode is at least one selected from the group consisting of gallium-indium alloy, gallium-indium-stannum alloy, bismuth-stannum alloy, and bismuth-stannum-plumbum-indium alloy. In some particular embodiments, a thickness of the liquid metal electrode is 500 nm to 2000 nm.

In some particular embodiments, a size of the circuit board connection pattern is 1 mm to 10 mm. In some particular embodiments, a width of the wire is 50 µm to 1000 µm. In some particular embodiments, a distance d between the electrode contacts is 3 mm to 50 mm.

In some particular embodiments, an insulating layer 210 and a skin adhesive layer 220 are provided on the flexible substrate 110 at positions other than the circuit board connection patterns 110 and the electrode contacts 130. The material of the insulating layer 210 includes at least one of silicon nitride, polydimethylsiloxane, and SU-8 photoresist.

The embodiments of the present disclosure further provide a preparation method for the above electromyographic electrode array. The preparation method includes the following steps:
(1) coating a liquid metal ink onto a substrate material to form a liquid metal electrode pattern, wherein the liquid metal electrode pattern includes a wire, a circuit board connection pattern disposed at an end of the wire, and an electrode connection pattern disposed at the other end of the wire;
(2) transfer-printing the liquid metal electrode pattern onto a flexible substrate to form several liquid metal electrodes; and
(3) performing plasma cleaning on the electrode connection pattern by a mask plate to change hydrophilicity or hydrophobicity of position of the electrode connection pattern, and dripping raw material of electrode contact to form several electrode contacts.

Referring to Figure 3, which shows a pattern mask plate of a liquid metal electrode used in some specific embodiments, the pattern mask plate includes a circuit board connection mask pattern 310 corresponding to the circuit board connection pattern 110, a wire mask pattern 320 corresponding to the wire 120, and a contact mask pattern 330 corresponding to the electrode contacts 130. The liquid metal ink is printed onto the pattern mask plate to form a liquid metal electrode pattern.

In some particular embodiments, a flexible substrate material is continuously coated on the pattern mask plate printed to form the liquid metal electrode pattern, the flexible substrate material is peeled off after being dried and solidified to form the flexible substrate, thus transferring the liquid metal electrode pattern onto the flexible substrate to form several liquid metal electrodes. In order to ensure the integrity and accuracy of the pattern, screen printing can serve as the printing method.

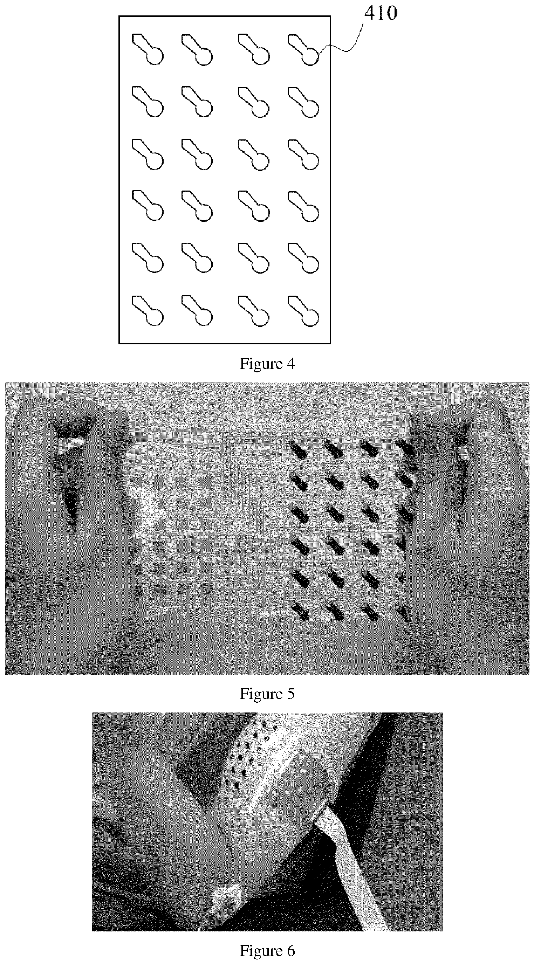

Referring to Figure 4, which is a schematic diagram of an electrode contact mask plate used in the preparation of an electrode contact in some embodiments, the electrode contact mask plate is provided with several through holes 410 having the same shape as the electrode contact pattern. The electrode contact mask plate is covered onto the flexible substrate printed with the liquid metal electrode pattern, and plasma cleaning is performed thereon, thus changing the hydrophilicity or hydrophobicity of the surface at the position of the electrode contacts. After peeling off the electrode contact mask plate, contact solution prepared from the raw material of the electrode contact is dropped at the position. Due to the difference in hydrophilicity caused by plasma cleaning, the contact solution will automatically form the required electrode contact pattern, and the electrode contact will be formed after drying.

In some particular embodiments, a process of the encapsulation of the flexible electromyographic electrode array is further performed after forming the electrode contact. The encapsulation process includes covering the electrode contacts and the circuit board connection pattern with a baffle, performing encapsulation, and removing the baffle. In some preferred embodiments, the specific way of encapsulation includes performing encapsulation on the flexible substrate to form an insulating layer and a skin adhesive layer. In some preferred embodiments, an insulating material is used for primary encapsulation, a skin adhesive is used for secondary encapsulation after primary encapsulation, and the baffle is removed after encapsulation. Further, the electrode contact and the circuit board connection pattern are covered with the baffle, a PDMS solution (including PDMS and an appropriate amount of curing agent) is used for spin coating and encapsulation; after the spin coating is completed, the flexible electromyographic electrode array is dried to complete the primary encapsulation; then a flexible skin adhesive is used for spin coating and encapsulation; and after the spin coating is completed, the flexible electromyographic electrode array is dried to complete the secondary encapsulation, and the baffle is removed.

The present disclosure further provides an electromyographic signal collection apparatus, which includes the above flexible electromyographic electrode array. In some particular embodiments, the electromyographic signal collection apparatus further includes a flexible circuit board, which is connected through a circuit board connection pattern on the flexible electromyographic electrode array. In some particular embodiments, the electromyographic signal collection apparatus further includes a receiving instrument, which is electrically connected to the flexible circuit board to collect the collected electromyographic signal.

The present disclosure further provides an electrophysiological signal collection method, which uses the aforementioned flexible electromyographic electrode array to collect electrophysiological signal. The specific method includes covering the flexible electromyographic electrode array onto a skin surface of a subject, and acquiring a subcutaneous electromyographic signal.

The present disclosure will be further described below through specific examples.

### Example 1

A flexible electromyographic electrode array was provided in this example, with the preparation method as follows.
(1) 3 g of gallium-indium alloy was weighted and added to 1 mL of n-decanol, and then a resulting mixture is subjected to an ultrasonic treatment for 1 min by an ultrasonic cell disrupter at 20% amplitude, so that the gallium-indium alloy was converted into micro-nano particles under an ultrasonic action, so as to prepare a liquid metal ink.
(2) A pattern mask plate for liquid metal electrode of fully flexible surface electromyographic electrode was designed by Computer Aided Design (CAD). Referring to Figure 3, there were 24 contact mask patterns and circuit board connection mask patterns, where a side length of the contact mask patterns was 2.5 mm, a spacing therebetween was 15 mm; and a side length of the circuit board connection mask pattern was 5 mm, and a width of the wire mask pattern was 250 µm. The prepared liquid metal ink was printed into liquid metal electrode patterns on a polyethylene terephthalate (PET) film by a screen printing method with the mask plate.
(3) After the printed liquid metal electrode pattern was dried by baking for 2 min at 80 °C, PDMS (polydimethylsiloxane, purchased from Dow Corning with a brand name of Sylgard 184) solution (a mass ratio of PDMS to a curing agent was 10: 1) was spin-coated on the liquid metal electrode pattern, and the thickness of PDMS was controlled to be 50 µm. The liquid metal electrode pattern was baked for 20 min again at 80 °C, and peeled off after the PDMS solution was solidified; and the liquid metal electrode pattern was transfer-printed onto a PDMS flexible substrate.
(4) 0.115 g of polyvinyl alcohol was dissolved in 5 mL of poly(3,4-ethylenedioxythiophene)/polystyrene sulfonate solution (PEDOT: PSS, 1.15 w/v%, a mass ratio of the polyvinyl alcohol to the PEDOT: PSS was approximately 2:1), 5% (v/v) dimethyl sulfoxide was added, and a resulting mixture was stirred for 4 h at 80 °C until a uniform and agglomeration-free PEDOT: PSS-PVA solution with good conductivity was obtained.
(5) A silicone sheet (polydimethylsiloxane) and a paper cutter were used to make an electrode contact mask plate (refer to Figure 4). The electrode contact mask plate was affixed to the electrode connection pattern position of the liquid metal electrode pattern, so that the through holes on the electrode contact mask plate correspond to the electrode connection pattern, and then plasma cleaning was performed. Due to the difference in hydrophilicity caused by plasma cleaning, the hydrophilicity and hydrophobicity of the electrode connection pattern position in the through holes were different from those around. After the mask plate was peeled off, the dripped PEDOT: PSS-PVA solution would automatically form the required electrode contact pattern. The amount of the PEDOT: PSS-PVA solution was controlled at 0.5 microliters per square millimeter to form a uniform conductive polymer film. After drying at 80 °C for 10 min, a flexible electromyographic electrode array with excellent conductivity and flexibility was obtained.
(6) A silicone sheet and a paper cutter were used to make baffles for encapsulating the electrode and the flexible circuit board connection point. The baffles were attached one by one to the electrode contact and the circuit board connection point pattern. The PDMS solution (a mass ratio of PDMS to a curing agent was 10:1) was used for spin coating and encapsulation, with a rotation speed being controlled at 2000 rpm and a rotation time of 30 s. After the spin coating was completed, the flexible electromyographic electrode array was baked at 80 °C for 10 min to complete a primary encapsulation. Then, a flexible skin adhesive (a mass ratio of component A to component B was 1:1, purchased from Dow Corning with a brand name of MG-9850) was used for spin coating and encapsulation, with the rotation speed being controlled at 2000 rpm and the rotation time of 30 s. After the spin coating was completed, the flexible electromyographic electrode array was dried at 80 °C for 10 min to complete a secondary encapsulation. The final product of the flexible electromyographic electrode array was then obtained by removing all the baffles with a tweezer, referring to the picture shown in Figure 5. Except for the electrode contacts and the circuit board connection point patterns, the rest of the product are sticky due to the presence of the flexible skin adhesive, so as to ensure continuous and stable contact among the device, the skin and the flexible circuit board.

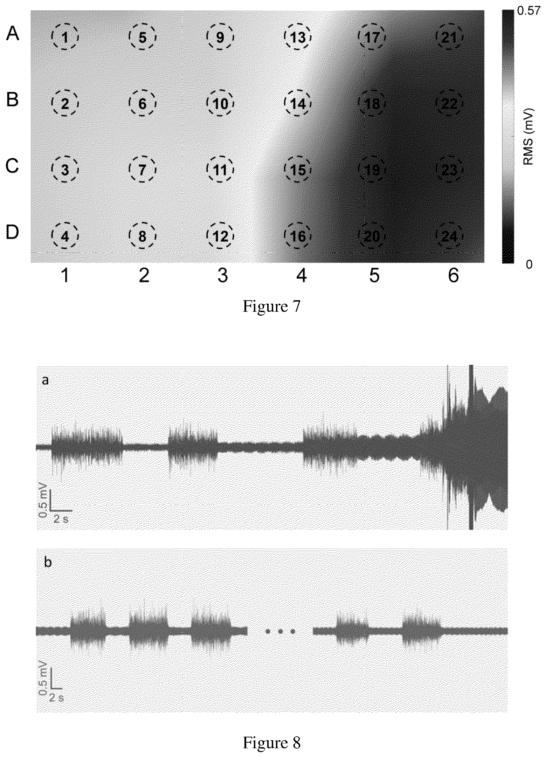

Referring to Figure 6, the flexible electromyographic electrode array was attached to the biceps brachii. The flexible electromyographic electrode array transmitted the electromyographic signal to a receiving instrument through the contact between the circuit board connection pattern and the flexible circuit board, so that the electromyographic signal produced by the biceps brachii during contraction could be obtained. Referring to Figure 7, through multi-channel signal collection by the electrode contacts 1 to 24 in the array, specific information on muscle fiber activity and tendon extension could further be obtained therefrom.

### Comparative Experiment

Comparative Example 1: a commercial electrode (US 3M, 2228) was provided, and the difference of the commercial electrode as compared to Example 1 was that the electrode contact was made of Ag/AgCl.

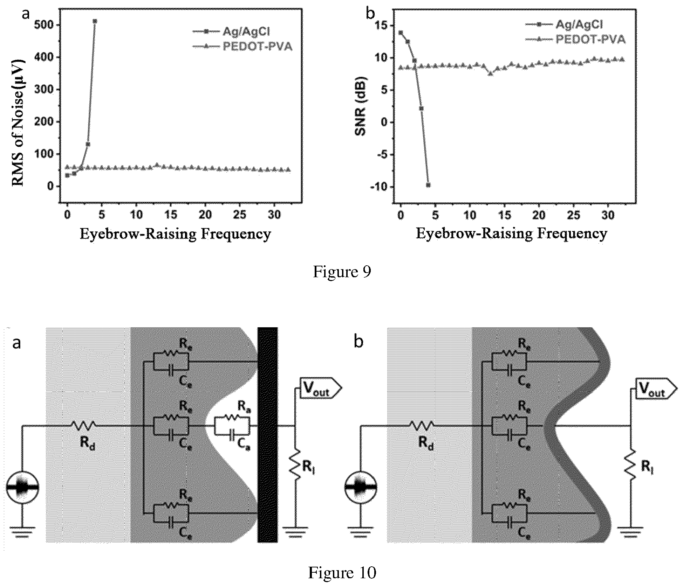

The flexible electromyographic electrode array provided in Example 1 and Comparative Example 1 were tested on frontalis muscle. One of the liquid metal electrodes and electrode contacts was used for the comparative experiment. The ground electrode of the flexible electromyographic electrode array was placed on the middle bone of the forehead, and the commercial electrode of Comparative Example 1 and the flexible electromyographic electrode array were placed on the left and right frontalis muscles respectively to conduct the frontalis muscle test. Electromyographic monitoring was bipolar monitoring (signals obtained from the working electrode and the ground electrode were differentiated), and the sampling rate was 1200 Hz. The subject performed an eyebrow-raising movement. The collected electrical signals were filtered by a 20Hz high-throughput filtering, and the results were shown in Figure 8, where panel a showed the result from Comparative Example 1, and panel b showed the result from Example 1. In Figure 9, panel a showed the RMS value of noise in the electrical signal, and panel b showed the signal-to-noise ratio of the electrical signal, where the signal-to-noise ratio = 20 × logic (RMS _{signal}/RMS ₙₒᵢₛₑ). Referring to the experimental results in Figures 8 to 9 and the principle shown in Figure 10 (panel a showed the principle for Comparative Example 1, and panel b for Example 1), it was found that after only 4 eyebrow-raising movements, the electrode of Comparative Example 1 generated additional capacitance and resistance due to loss of flexibility and a gap on the skin, so that the overall circuit impedance was increased, and the signal was covered by the increased noise value, leading to an incapability of accurate measurement. In comparison, the electrode provided in Example 1 could be perfectly embedded in the skin folds, and after 33 eyebrow-raising movements (5 min of duration), stable baseline noise was still maintained, thus accurately reading signals.

### Example 2

A flexible electromyographic electrode array was provided in this example, which differed from Example 1 in that an equal mass of polyethylene oxide was used to replace polyvinyl alcohol. Using the method in the Comparative Experiment for detection, it was found that the stable baseline noise was still maintained after multiple eyebrow-raising movements, and the signal could be read accurately.

### Example 3

A flexible electromyographic electrode array was provided in this example, which differed from Example 1 in that an equal mass of polyethylene glycol was used to replace polyvinyl alcohol. Using the method in the Comparative Experiment for detection, it was found that the stable baseline noise was still maintained after multiple eyebrow-raising movements, and the signal could be read accurately.

### Example 4

A flexible electromyographic electrode array was provided in this example, which differed from Example 1 in that 0.115 g of polyvinyl alcohol was dissolved in 1 mL of poly(3,4-ethylenedioxythiophene)/polystyrene sulfonate solution (PEDOT: PSS, 1.15 w/v%). Using the method in the comparative experiment for detection, it was found that the stable baseline noise was still maintained after eyebrow-raising movements, and the signal could be read accurately.

### Example 5

A flexible electromyographic electrode array was provided in this example, which differed from Example 1 in that 0.115 g of polyvinyl alcohol was dissolved in 40 mL of poly(3,4-ethylenedioxythiophene)/polystyrene sulfonate solution (PEDOT: PSS, 1.15 w/v%). Using the method in the comparative experiment for detection, it was found that the stable baseline noise was still maintained after eyebrow-raising movements, and the signal could be read accurately.

### Example 6

A flexible electromyographic electrode array was provided in this example, which differed from Example 1 in that the spacing of the contact mask patterns was 5 mm, so that the spacing of electrode contacts in the finally prepared electrode array was 5 mm. Using the method in the comparative experiment for detection, it was found that the stable baseline noise was still maintained after eyebrow-raising movements, and the signal could be read accurately.

### Example 7

A flexible electromyographic electrode array was provided in this example, which differed from Example 1 in that the spacing of the contact mask patterns was 30 mm, so that the spacing of electrode contacts in the finally prepared electrode array was 30 mm. Using the method in the comparative experiment for detection, it was found that the stable baseline noise was still maintained after eyebrow-raising movements, and the signal could be read accurately.

The present disclosure has been described in detail above with reference to the embodiments. However, the present disclosure is not limited to the above-mentioned embodiments. Various changes can be made within the knowledge scope of those of ordinary skill in the art without departing from the purpose of the present disclosure. In addition, the embodiments of the present disclosure and the features in the embodiments may be combined with each other without conflict.

## Claims

1. A flexible electromyographic electrode array, comprising:
a flexible substrate;
several liquid metal electrodes provided on the flexible substrate, wherein each of the liquid metal electrodes comprises a wire and a circuit board connection pattern disposed at an end of the wire; and
several electrode contacts provided on the flexible substrate, wherein each of the electrode contacts is connected to the other end of the wire, and raw material of the electrode contact comprises a hydrophilic polymer and poly(3,4-ethylenedioxythiophene)/polystyrene sulfonate.

2. The flexible electromyographic electrode array according to claim 1, wherein a mass ratio of the hydrophilic polymer to the poly(3,4-ethylenedioxythiophene)/polystyrene sulfonate is 1: (0.1 to 4).

3. The flexible electromyographic electrode array according to claim 1, wherein the raw material of the electrode contact further comprises at least one of dimethyl sulfoxide, ethylene glycol, and glycidyloxypropyltrimethoxysilane.

4. The flexible electromyographic electrode array according to claim 1, wherein the hydrophilic polymer is at least one selected from the group consisting of polyethylene glycol, polyethylene oxide, and polyvinyl alcohol.

5. The flexible electromyographic electrode array according to any one of claims 1 to 4, wherein a skin adhesive layer is further provided on the flexible substrate.

6. The flexible electromyographic electrode array according to any one of claims 1 to 4, wherein a spacing between the electrode contacts is 3 mm to 50 mm.

7. A preparation method for the flexible electromyographic electrode array according to any one of claims 1 to 6, comprising the following steps:
(1) coating a liquid metal ink onto a substrate material to form a liquid metal electrode pattern, wherein the liquid metal electrode pattern comprises a wire, a circuit board connection pattern disposed at an end of the wire, and an electrode connection pattern disposed at the other end of the wire;
(2) transfer-printing the liquid metal electrode pattern onto a flexible substrate to form several liquid metal electrodes; and
(3) performing plasma cleaning on the electrode connection pattern by a mask plate to change hydrophilicity or hydrophobicity of position of the electrode connection pattern, and dripping raw material of the electrode contact to form several electrode contacts.

8. The preparation method according to claim 7, further comprising step (4): covering the electrode contacts and the circuit board connection pattern with a baffle, performing encapsulation, and removing the baffle.

9. An electromyographic signal collection apparatus, comprising the flexible electromyographic electrode array according to any one of claims 1 to 6.

10. An electrophysiological signal collection method, comprising covering the flexible electromyographic electrode array according to any one of claims 1 to 6 onto a skin surface of a subject, and acquiring an electromyographic signal.
